(19) Europäisches Patentamt

European Patent Office

Office européen des brevets

(11) EP 0 893 690 B1

(12) EUROPEAN PATENT SPECIFICATION

(45) Date of publication and mention
of the grant of the patent:
**14.07.2004 Bulletin 2004/29**

(51) Int Cl.⁷: **G01N 33/543**, G01N 33/569

(21) Application number: **97870123.3**

(22) Date of filing: **19.08.1997**

(54) **Detection of mycotoxins by flow-through membrane-based enzyme immunoassay**

Nachweis von Mycotoxinen mittels membran-gebundenen Durchfluss-Enzymimmunoassays

Détection des mycotoxines par immunoessai enzymatique à flux traversant sur une membrane

(84) Designated Contracting States:
**AT BE DE DK ES FI FR GB GR IE IT LU NL PT SE**

(30) Priority: **14.07.1997 EP 97870104**

(43) Date of publication of application:
**27.01.1999 Bulletin 1999/04**

(73) Proprietor: **Toxi-Test N.V.**
**9990 Maldegem (BE)**

(72) Inventors:
• **De Saeger, Sarah**
**9300 Aalst (BE)**

• **Van Peteghem, Carlos**
**9840 De Pinte (BE)**

(74) Representative:
**Brants, Johan Philippe Emile et al**
**De Clercq, Brants & Partners cv**
**Edgard Gevaertdreef 10a**
**9830 Sint-Martens-Latem (BE)**

(56) References cited:
**EP-A- 0 402 023** **EP-A- 0 508 010**
**EP-A- 0 537 827** **WO-A-85/05451**
**WO-A-86/02736** **DE-A- 4 013 004**
**GB-A- 2 193 809**

**Description**

The detection of mycotoxins by a flow-through membrane-based enzyme immunoassay is described. The test device consists of a plastic top and bottom member that fit together. The bottom member contains the absorbent material. The top member has a round opening. A microporous membrane coated with immunoreagents is placed on the absorbent material. All reagents are dropped onto the membrane through the opening of the top member. Due to the contact between the membrane and the absorbent material, added reactants are drawn through the membrane into the absorbent material a second antibody technique is used. The membrane is coated with anti-mouse antibodies. The following reactants are successively dropped onto the membrane : wash solution, monoclonal anti-mycotoxin antibodies, wash solution, sample, enzyme labelled mycotoxin and wash solution. Finally, substrate solution is added to cause a colour development. This test is used for the detection of mycotoxins in food and feed.

FIELD OF THE INVENTION

This invention relates to an assay for an analyte, and more particularly to a flow-through membrane-based enzyme immunoassay for mycotoxins in food and feed.

BACKGROUND OF THE INVENTION

Mycotoxins are toxic secondary metabolites of low molecular weight produced by moulds during their growth on food and feed. Ingestion of such compounds can result in toxic syndromes in humans and animals. Human ingestion of mycotoxins will mainly occur from the consumption of mycotoxins in plant-based foods and the residues and metabolites in animal-derived foods such as milk, cheese, and some meat products. As a consequence of their diversity of chemical structures and physical properties, individual mycotoxins are different in the degree and manner of toxicity. Mycotoxicoses are possibly the most unfamiliar and least investigated of the diseases that affect humans and animals. While at least 300 mycotoxins have been identified at laboratory level, only about 20 of these occur naturally in food and feed at significant levels and frequency to be of food safety concern. Some important naturally occurring mycotoxins, the toxin producing moulds, the major toxic effects in humans and animals and their typical natural substrates are shown in Table 1 (p. 27).

Contamination of foods and feeds with mycotoxins is very common and difficult to control. It is a worldwide problem. The UN Food and Agriculture Organisation has estimated that up to 25% of the world's foods are significantly contaminated with mycotoxins. Mycotoxin production can occur in the field, during harvest, processing, storage and transport. The detectable presence of moulds in food does not automatically indicate that mycotoxins have been formed. On the other hand, the absence of viable moulds in foods does not necessarily mean there are no mycotoxins. Moulds could be killed during food processing, but mycotoxins could persist. They are relatively heat-resistant and very difficult to destroy. To decrease the risk of exposure for humans and animals and to minimize economic losses, several countries have set tolerance levels for mycotoxins in foods and feeds. An overview has been published by van Egmond, H.P. (1989. Food Addit. Contam. 6:139-188). Most of the existing regulations concern the aflatoxins. There are also some countries with regulations for ochratoxin A (OA), trichothecenes, patulin, zearalenone and fumonisins. However, there is no harmonisation of mycotoxin regulations in the different countries. Maximum tolerated levels for OA e.g. range from 1 to 50 $\mu$g/kg for food and from 100 to 1000 $\mu$g/kg for animal feed. Therefore, the European Commission for example makes efforts towards an harmonisation between the different Member States. So, the European Commission has proposed a maximum permitted level of 4 $\mu$g/kg for OA in cereals.

To control these regulations, it is necessary that the amount of mycotoxins in food and feed can be measured. Many analytical methods exist for mycotoxins in food and feed. In general, the suspected contaminated commodity is first subjected to a rigorous sampling program. This is very important because mycotoxins are often unevenly distributed in the commodity. Once a representative sample is obtained, it is necessary to extract the toxin from the complex matrix for subsequent analysis. Interfering co-extracted substances will be removed by a clean-up treatment. In the actual analysis, methods for separation, detection and quantification are included in a single step. In addition, a confirmatory test must be included to assure true identity of the toxin. An overview of mycotoxin analysis has been given by Chu, F.S. (1995. Mycotoxin Analysis, p. 283-332. In Analyzing Food for Nutrition Labeling and Hazardous Contaminants. I. J. Jeon and W.G. Ikins (eds.). Marcel Dekker, Inc., New York). Because of the diversity of chemical structures and physicochemical properties of mycotoxins, there is no uniform analytical method for the different mycotoxins, and neither for a specific toxin in various foods and feeds.

First, bioassays can be used for mycotoxin analysis. Laboratory animals are exposed to the toxins. Either death, pathological lesions, biochemical events or immunotoxic effects are used as an index for toxicity. Such biological methods are simple, but not specific. The sensitivity is low when compared with other methods. Bioassays can be used as a tool for screening for general toxicity. They can complement chemical analysis and may warn of the presence of a toxin even when its chemical identity is unknown.

Secondly, chemical methods are used for mycotoxin analysis. Most quantitative methods for mycotoxins use a final chromatographic separation followed by a suitable detection step.

Thin-layer chromatography (TLC) is one of the earliest analytical methods developed for mycotoxin analysis and it is one of the most commonly used methods for mycotoxin analysis. It is also used as a multi-mycotoxin screening technique. This means a simultaneous analysis of several mycotoxins. However, extensive sample clean-up is necessary and experienced personnel is needed. TLC is a slow technique and is not suitable for automation.

High performance liquid chromatography (HPLC) is increasingly used for mycotoxin analysis due to its higher sensitivity and improved accuracy compared with TLC. However, when the mycotoxin does not contain fluorescent groups or does not show UV absorption, derivatization is necessary to make UV or fluorescent detection possible. A rigorous clean-up of the samples is needed before injection onto the HPLC column.

Gas-liquid chromatography (GLC) is not very commonly used for mycotoxin analysis. This method is only used for detection of mycotoxins with no or low fluorescence or low absorptivity (e.g. trichothecenes). Sample clean-up is needed and polar groups must first be converted to their esters/ethers before GLC analysis. Flame ionization and electron capture detection are used. Confirmation of mycotoxins in GLC analysis is generally carried out by mass spectrometric methods.

Chromatographic procedures are time consuming. It takes several hours to several days to complete the analysis. The equipment used is expensive and experienced technicians are needed.

Thirdly, there is very much interest in analyzing mycotoxins by immunochemical methods. Immunoassays overcome many of the main analytical problems. They are highly specific, sensitive, simple to operate and require little or no capital outlay.

Large numbers of samples can be tested in one assay and only little sample preparation is required. Immunoassays are screening methods. So, every positive result must be confirmed by another method. Immunochemical techniques are based upon a reversible binding between an antigen and an antibody which is specific for the antigen. An antibody (or immunoglobulin) is a binding protein which is synthesized by the immune system of an animal in response to either the invasion of a foreign organism or the injection of an immunogenic antigen. Laboratory animals such as rabbits and mice are used to make antibodies against specific substances.

These antibodies are exploited in immunochemical techniques for the detection of the original antigenic substance. Antibodies are divided into different classes. The principal one usually employed in an immunoassay is immunoglobulin G (IgG).

Various immunological assays have been developed. Radioimmunoassay (RIA) and enzyme-linked immunosorbent assay (ELISA) are the most important for mycotoxin analysis.

In a RIA the specific antibody (Ab) is simultaneously incubated with a solution of unknown sample (Ag) or mycotoxin standard (Ag) and a constant amount of toxin labelled with a radioisotope (Ag*).

$$Ab + Ag + Ag^* \quad \rightarrow \quad Ab\text{-}Ag + Ag^*$$

$$Ab\text{-}Ag^* + Ag$$

The labelled antigen will be prevented from binding to the antibody to an extent directly proportional to the concentration of the antigen in the solution. After separation of the free and bound toxin, the radioactivity in these fractions is determined. The toxin concentration of the unknown sample is determined by comparing the results to a standard curve, which is established by plotting the ratio of radioactivities in the bound fraction and free fraction vs. log concentration of unlabelled standard toxin.

Although RIAs provide accurate data for mycotoxin analysis, there are much disadvantages. The use of radioisotopes is a potential health hazard. The isotopes that are most commonly used, have a short shelf life. This means that the labelled component must be periodicity replaced by new product. Furthermore, there is the problem of disposal of radioactive waste and the equipment required for measuring radioactivity is expensive.

For these reasons, alternative labels have been used e.g. enzymes in enzyme immunoassays. These techniques are much safer and cheaper. There are several variations of enzyme immunoassays. ELISAs have been successfully applied to the detection of mycotoxins in food and feed. It is based on the same principles as RIA except that enzyme activity is measured instead of radioactivity. Solid-phase systems are used for the separation of free and bound components. Antibody or antigen is coupled to the solid support. In most cases the solid phase is a microtiter plate. An antibody immobilized on the microtiter wells, will, when it is exposed to a solution containing the antigen, bind the antigen and render it in turns immobilized. All non-reacted components can be simply washed away. A covalent conjugate of an antibody or an antigen and an enzyme is another essential component of an ELISA.

When an antigen enzyme conjugate binds to an immobilized antibody, its presence can be detected through the activity of the coupled enzyme, e.g. by adding an enzyme substrate which will be transformed into a coloured product.

A microtiter plate ELISA consists in successive incubation and washing steps. So, several hours are required to complete the test. Other drawbacks of microtiter plate immunoassays are the need for sophisticated equipment and qualified personnel, and the restriction of the application to laboratories.

The most common method for immobilization of antibodies or antigens on the microtiter plate is passive adsorption. However, it has been reported that passive adsorption of proteins on hydrophobic surfaces can produce substantial conformational and functional changes in proteins (Butler, J.E., et al. 1993. Mol. Immunol. 30:1165-1175). To overcome this problem, surface-modified hydrophilic membrane supports that covalently bind proteins can be used. It has been proved that such membranes retain immobilized proteins in their native conformations (Pfund, W.P., and J.S. Bourdage. 1990. Mol. Immunol. 27:495-502). Microporous membranes provide an easy to manipulate support or carrier for the immobilized antigen or antibody.

In recent years, diagnostic testing in the agricultural and veterinary fields has been expanding to alternate testing sites, such as farms, storehouses, and factories. To meet this market's need, the complex multistep immunoassay has to be compressed into a self-contained package. This requires novel approaches. The techniques should be simple, should use samples without manipulation or with minimal manipulations, and must provide accurate results with little or no instrumentation. Inspection services can use rapid, cheap and easy tests to check the compliance with the mycotoxin regulations. Producers will also benefit from the new technology : ready and easy to use tests will enable them to check their products prior to sale.

Microporous membrane substrates are valuable solid phases in immunoassays for application in the field. Many of those membrane-based field tests have been described e.g. flow-through membrane-based enzyme immunoassays. In such assays, the membrane coated with immunoreagents, is placed on an absorbent layer. Due to the flow-through characteristics of the microporous membrane, added reactants are drawn through the membrane because of its contact with the absorbent bed. This brings the immunoreagents rapidly into close contact with the reactive sites on the membrane. The kinetics of reactions between immobilized and soluble reactants closely approach the kinetics of binding in solution. As a consequence, assay time will be very short.

Many flow-through devices and assays have been disclosed. For example, U.S. Patent No. 4,246,339 (Cole et al.) describes a portable immunochemical test device for testing liquid samples such as biological fluids for the presence of a diagnostic reagent. The device comprises telescoping top and bottom members defining a liquid reservoir therebetween. The top member has one or more test wells each having a bottom opening covered with a microporous membrane, the membrane having fluid passages therethrough and having a co-reactant (immunoreagent) immobilized to its internal and external surfaces. The device comprises resilient means for biasing the members in the open position and sorbent material in the reservoir between the members spaced from the membrane in the open position but in contact therewith in the closed position. Liquids are passed through the membrane into the sorbent material by depressing the members to the closed position.

U.S. Patent No. 4,407,943 (Cole et al.) describes a diagnostic assay method for the detection of antigen or antibody in body fluids, exploiting immunochemical reactions. Therefore antigen or antibody are immobilized on a zein- or collagen-coated microporous membrane. In a second stage, an immunochemically neutral protein is immobilized on the membrane to minimise nonspecific binding during the immunoassay. The fluid to be tested is allowed to flow through the pores of the membrane. Absorbent material is not used. As in a conventional ELISA there is, at the end of the test, a colour development which is dependent on the amount of component present in the test fluid. This assay is used for the detection of antibody to Toxoplasma gondii in serum.

U.S. Patent No. 4,632,901 (Valkirs et al.), U.S. Patent No. 4,727,019 (Valkirs et al.), U.S. Patent No. 5,120,504 (Petro-Roy et al.), EP 0253579 (Petro-Roy et al.) and WO 85/05451 (Valkirs et al.) Valkirs, G.E., and R. Barton (1985. Clin. Chem. 31:1427-1431) describe a process for simply and rapidly performing immunoassays which uses a simple apparatus and which does not require lengthy incubation steps. The apparatus comprises firstly a membrane to which is covalently bound e.g. an antibody and secondly absorbent material which acts when in contact with the membrane to induce flow through the membrane when a fluid sample is added. Membrane and absorbent layer are enclosed in a container open at one end to permit sample to be applied to the membrane. All types of ELISA can be performed with this apparatus (e.g. competitive assay, sandwich assay etc...). This test is used for clinical applications.

U.S. Patent No. 5,240,844 (Wie et al.) describes a colorimetric testing device to determine the presence of a specific substance in a liquid medium. The testing device has the size of a plastic credit card and can be used outside the laboratory. The test card includes top and bottom sheets adhesively secured to an intermediate frame member which defines a filter chamber having a flat filter therein to which antibodies are bound. In the top sheet there are test and control ports through which the other immunoreagents can be administered. The EZ-Screen® (Editek, Inc., Burlington, NC, USA) tests for the detection of aflatoxins, OA, zearalenone and T-2 toxin are covered by this patent.

Also EP 0302673 (Lovell et al.), EP 0334015 (Gary et al.), EP 0402023 (Chu et al.), EP 0458198 (Clark), EP 0537827 (Contestable et al.) and EP 0444303 (McLaurin et al.) describe other variations on flow-through assays and devices. They are used for assaying biological samples. GB 2193809 describes mycoloxins assay reagent and assay method.

Two papers published by Schneider et al. describe flow-through tests for food analysis. One paper (1994. Arch. Lebensmittelhyg. 45:43-45) describes a flow-through test ("immunofiltration test") for the detection of chlóramphenicol residues in raw milk. The other paper (1995. J. Agric. Food Chem. 43:2548-2552) decribes an enzyme-linked immunofiltration assay for the rapid detection of fumonisin B1 in corn-based food. As mentioned before, fumonisin B1 is one of the mycotoxbs. A third paper (1995, Food Additives and Contaminants, 12, 3: 405-413) discloses a device for performing an immunofiltration assay for the detection of paralytic shellfish poisoning toxius.

However in contrast with their test, we use single-well tests, a second antibody technique and a new surface-modified hydrophilic membrane support.

OBJECTS OF THE INVENTION

The principal object of the present invention is to provide a membrane-based field test for the detection of mycotoxins in food and feed. More specifically, a flow-through membrane-based enzyme immunoassay for the qualitative determination of mycotoxins has been developed.

A further object is the provision of a test that can be visually evaluated, i.e. without the need of instruments. Another object includes the provision of a simple test which can be performed in less than 15 minutes by less qualified personnel and outside the laboratory, at the location where samples are taken.

An additional object of our invention is the provision of a test which will use little quantities of reagents which results in Lower costs and less (toxic) waste.

Another important object is to provide a test of the aforementioned character with a sensitivity in compliance with the mycotoxin regulations. For OA for example, it has been the aim to develop a test with a sensitivity of 4 μg/kg for cere-als (4 μg/kg is the maximum permitted level proposed by the European Commission).

Other and additional objects and advantages will be apparent from the following description.

SUMMARY OF THE INVENTION

The invention relates to a flow-through immunoassay as described in claim 1 for detecting a mycotoxin (19) chosen from the group of mycotoxins comprising aflatoxins, citrinin, fumonisins, ochratoxins, patulin, trichothecenes and zearalenones, using a container containing an absorbent material and having an opening closed by a microporous surface-modified hydrophilic membrane contacting the absorbent material and having pores with a size tower than 1 μm and which has a first part that is coated with a first antibody (16) which is immunologically reactive with an antibody (18) against the mycotoxin (19) to be detected and a second part on which proteins different from the first antibody are immobilized, and whereby the assay comprises the following steps in order of execution, whereby a subsequent step is only executed after the solution used in the previous step is adsorbed by the absorbent material (13):

- flowing through the membrane (14) of a wash solution.
- flowing through the membrane of a solution of an antibody (18) against the mycotoxin to be detected,
- flowing through the membrane (14) of a wash solution so as to flush from the membrane antibody (18) which is not bound to the first antibody (16) on the membrane,
- flowing through the membrane (14) of the sample extract solution.
- flowing through the membrane of a solution of an enzyme-labeled antigen (20) which is a conjugate of the mycotoxin to be detected with an enzyme.
- flowing through the membrane (14) of a wash solution,
- flowing through the membrane (14) of a solution (21) of a substrate-chromogen causing a color development upon reaction with the enzyme.

Advantageously the membrane has pores with a size of between 0.3 μm and 0.7 μm. Preferably, the membrane has pores with a size of about 0.45 μm. According to an embodiment, the porosity of the membrane represents more than 60% of the total volume of the membrane. Preferably, the porosity of the membrane represents more than 70% of the total volume of the membrane. Other preferred embodiments are further defined in claims 2 to 4.

The invention relates also to a test kit as described in claim 5, for performing a flow-through immunoassay for detecting a mycotoxin (19) chosen from the group of mycotoxins comprising aflatoxins, citrinin, fumonisins, ochratoxins, patulin, trichothecenes and zearalenones, in a sample extract solution, said test kit comprising at least:

- a device comprising a bottom member (11) forming a reservoir comprising absorbent material (13) and a top member (10) which fits the bottom member (11) and which presents a single opening (12) through which reagents can be dropped,

- a microporous surface-modified hydrophilic membrane (14) placed on the absorbent material, said membrane having pores with a size lower than 1 μm and which is coated with a first antibody (16) which is immunologically reactive with an antibody (18) which has been raised against the mycotoxin to be detected,
- a wash solution,
- an assay buffer,
- a solution of the antibody (18) against the mycotoxin to be detected,
- a solution of enzyme-labeled antigen (20), which is a conjugate of the mycotoxin to be detected with an enzyme,
- a solution of substrate-chromogen which causes a color development upon reaction with the enzyme, and
- a negative control.

Preferred embodiments are as further described in claims 6 and 7.

The invention relates further to the use of a test kit as described in claim 8 for detecting Ochratoxin A in a sample extract wherein in said test kit, the solution (18) is a solution of the antibody against Ochratoxin A and the solution (20) is a solution of enzyme-labeled Ochratoxin A (20), which is a conjugate of Ochratoxin A with an enzyme.

The present invention provides thus a flow-through membrane-based enzyme immunoassay for the detection of mycotoxins in food and feed. As an example the flow-through test for OA in wheat will be described. However, the invention is not limited thereto.

The test device or container consists for example of a plastic top and bottom member that fit together. These members are impervious to liquids. The top member has a round opening with raised edges. The bottom member is the liquid receiving reservoir. It contains the absorbent material. A microporous membrane coated with immunoreagents is placed on the absorbent material. When the top member is placed on the bottom member, it presses the membrane to the absorbent material. All reagents are dropped onto the membrane through the opening of the top member.

Due to the contact between the membrane and the absorbent material, added reactants are immediately drawn through the membrane into the underlying absorbent layer.

In a preferred format, a second antibody technique is used. A second antibody is an antibody raised against the immunoglobulins of the animal species in which the first antibody was raised. The first antibody is the antibody reacting specifically with the antigen being measured. In a second antibody technique the second antibody is immobilized on the microporous membrane.

The following reactants are successively dropped onto the prepared membrane : wash solution, first antibody, wash solution, sample, antigen-enzyme conjugate and wash solution. Between each step it is preferred to wait until the liquid is absorbed by the absorbent pad. The desired immunochemical reaction between the components in the added fluids and the components immobilized on the solid support, takes place during the period of time in which the fluid is in contact with the reactive sites on the membrane, i.e. during passage through the membrane. Incubation times are extremely short.

Finally, for example, a substrate-chromogen mixture specific to the enzyme is passed through the membrane to cause a colour development. There is an inverse relationship between antigen concentration and colour development. If no antigens are present in the sample, the colour development will be maximal. The colour intensity can be visually evaluated, i.e. with the naked eye. To confirm and quantify the visual observations, a portable colorimeter can be used.

This flow-through assay can be performed in less than 15 minutes.

BRIEF DESCRIPTION OF THE FIGURES

FIG. 1 is a picture of the different parts of the test device;
FIG. 2 is a drawing of a membrane section;
FIG. 3 illustrates the flow-through enzyme immunoassay;
FIG. 4 is a picture of the results of assaying a negative control solution and an OA solution of 0.4 ppb;
FIG. 5 is a dose-response curve of the flow-through enzyme immunoassay for the detection of OA in buffer solutions;
FIG. 6 is a dose-response curve of OA in buffer solution and in OA-free wheat extract;
FIG. 7 shows the results of the analysis of OA in wheat reference materials with the flow-through assay, and
FIG. 8 is a cross section of the test device.

DETAILED DESCRIPTION OF THE INVENTION

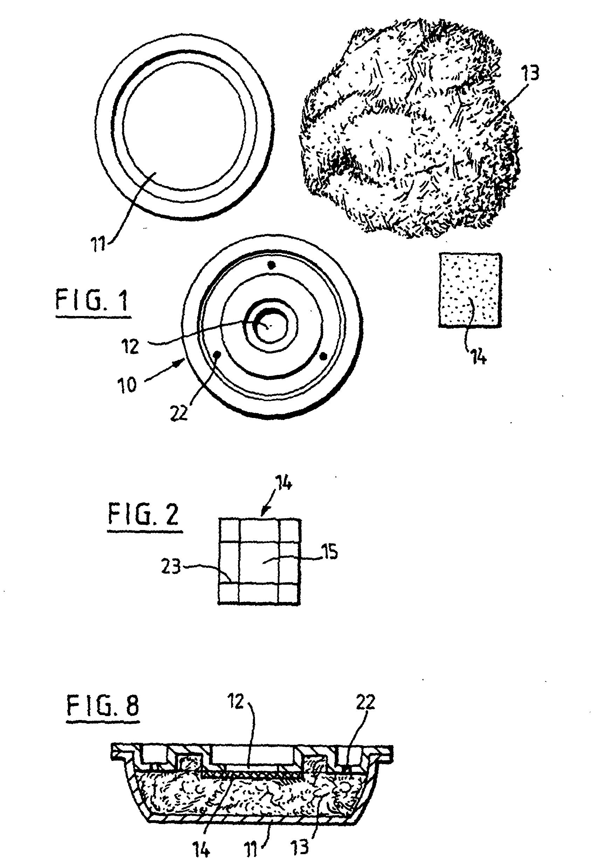

Referring to FIG. 1, the test device consists of a plastic top member 10 and a plastic bottom member 11. The top member 10 closely fits the bottom member 11. This snap-fit device can be obtained from Trosley Equipment, Dover, Kent, UK. After appropriate cleaning, this device can be reused. The top 10 and bottom member 11 are impervious to liquids. Top member 10 has a round opening 12 with raised edges.

The top member also comprises three little holes 22 for air passage. The bottom member 11 is the liquid receiving reservoir. Preferably, the plastic test device from Trosley Equipment, Dover, Kent, UK is used, but other formats with the same characteristics can be used too.

The bottom member 11 contains the absorbent material 13. A microporous membrane 14 coated with immunoreagents is placed on the absorbent material 13.

When the top member 10 is fitted on the bottom member 11, which is filled with the absorbent pad 13 and the microporous membrane 14, the membrane 14 is firmly pressed to the absorbent material 13. There is no disassembly of the test device during the assay. This very close contact between the membrane and the absorbent pad is a very important condition for having a successful flow-through assay. If test devices, membranes or absorbent materials different from those described here are used, that condition must be certainly fulfilled.

In conventional solid-phase immunoassays, one of the immunoreagents (e.g. antibodies) is immobilized on the solid support. The added immunoreagents (e.g. antigen) are in solution and must diffuse to the immobilized components. As a consequence, the kinetics of antibody-antigen reaction is slower than when both components are in solution and free to diffuse. With a flow-through immunoassay, one of the immunoreagents (e.g. the antibody) is also immobilized on a solid support.

But now, the solid support is a liquid permeable microporous membrane. The pores permit liquid flow through the membrane. When an absorbent layer is placed below the membrane, in close contact with the membrane, the added fluid reactants are actively drawn through the membrane into the absorbent means. The added reactants are really brought to the reactive sites on the membrane. The kinetics of antibody-antigen reaction approaches the kinetics of binding in solution. As a consequence, assay time will be very short in comparison with the conventional solid-phase immunoassays.

Another advantage of membranes over conventional solid phases is their higher protein binding capacity due to their porosity and consequential large surface area.

Higher protein binding capacity means increased mass action and therefore increased speed and sensitivity of an assay using that solid phase.

Membranes also offer flexibility in fabrication and design which makes it possible to modify the solid phase to meet the requirements of each individual assay.

However, a disadvantage is that proteins bind to many membranes through an hydrophobic, electrostatic or ionic interaction. This binding is indiscriminate and reversible. The sensitivity and reproducibility of a solid-phase immunoassay is dependent on the ability of the proteins to remain bound to the membrane.

Furthermore, passive adsorption of proteins on hydrophobic surfaces is an inactivation process: it can produce substantial conformational and functional changes in most of the adsorbed proteins. Surface-modified hydrophilic membrane supports that covalently bind proteins offer a major solution to these problems. A covalent bond is permanent and irreversible. Proteins bound to such membranes are retained in their native conformation. As a preferred membrane 14, the Immunodyne ABC membrane, purchased from Pall BioSupport, Portsmouth, England, is used. It is a nylon membrane which has been surface modified with reactive groups to covalently bind proteins and other compounds containing amino groups. The preferred pore size is 0.45 μm. The pores occupy from about 75 to 85% of the membrane volume. The Immunodyne ABC membrane provides a white surface, even when wet, for excellent colour discrimination.

All reagents are dropped onto the membrane 14 through the aperture 12 of the top member 10.

As absorbent material 13, 100% cotton wool is preferred. Each test device is filled with 1.5 g of cotton wool. This is enough to absorb all liquid passed through the membrane 14 and to enable a very close contact between the membrane 14 and the absorbent material 13. Due to this contact, flow of liquid through the membrane 14 will be induced by the absorbent member 13 without the use of external means, even when the hydrostatic pressure of the added reagents used in the assay is not enough to induce flow through the membrane.

The test device with the aforementioned character is used to perform an enzyme immunoassay. The assay for the detection of OA in wheat is used as a specific example to further demonstrate the invention. However, this example is not to be construed as limiting the invention to the specific procedure described in it.

Antibodies and conjugate. As previously described, an antibody reacting specifically with the antigen being measured and an enzyme labelled antigen are two essential components in an enzyme immunoassay. These immunoreagents are produced by the Institute for Animal Sciences, Agricultural Biotechnology Center, Gödöllö, Hungary.

The antigen being measured is the OA. The antibody against OA is a monoclonal antibody. Production of monoclonal antibodies involves different steps. At first mice are immunized with the immunogen. Mycotoxins (antigens) have low molecular weights. Therefore, they do not independently induce an immune response. They must be conjugated to a carrier protein (e.g. bovine serum albuminutes [BSA]) to be rendered immunogenic (immunogens). Furthermore splenocytes of the mice are fused with myeloma cells. Antibody-secreting hybridomas (= fusion products) are selected and cloned and ascites is produced. These techniques are well known to those skilled in the art. Details about the production and characterization of the monoclonal anti-OA antibody are given by Gyöngyösi-Horvath A. et al. (1996. Lett. Appl. Microbiol. 22:103-105). This anti-OA antibody is very specific. It shows a weak cross-reaction with ochratoxin B (9.3%). There is no cross-reaction with ochratoxin a, coumarin, 4-hydroxy-coumarin and D,L-phenylalanine. The anti-OA antibody is IgG1 with kappa light chains. The affinity constant is $7.8 \times 10^9$ M$^{-1}$. The anti-OA immunoglobulin fraction (protein content, 2.5 mg/ml) is used for the flow-through enzyme immunoassay. This immunoglobulin fraction is prepared from ascites by ammonium sulfate precipitation, followed by dialyzing against phosphate-buffered saline (PBS). This purification is well known to those skilled in the art.

Horseradish peroxidase (HRP) has been chosen as the enzyme for labelling the antigen. HRP is an oxidoreductase which transfers hydrogen from hydrogen donors to hydrogen peroxide. Different chromogenic substrates can act as hydrogen donors that form a coloured product on oxidation. HRP is very frequently used in immunoassays because of its stability, high catalytic rates and ease of conjugation to other molecules. Because OA possesses a free carboxylic group, it can be directly conjugated to HRP by a mixed anhydride method as described by Bama-Vetro I. et al. (1996. J. Agric. Food Chem. 44:4071-4074).

Preparation of membranes. The preferred membrane is cut into sections (2 by 2 cm) 14. Membranes must be handled using gloves to prevent membrane contamination by fingerprint residues. Cleaned scissors are used to cut the membrane. To enable reproducible cutting, lines are drawn on the membrane with a pencil. Proteins are immobilized on each membrane section in the form of a spot. The spotting can be done manually with a micropipette or automatically by using e.g. the CAMAG Automatic TLC Sampler (ATS3). Automatic spotting results in more reproducible spots but requires more time. The protein solution is spotted in the centre 15 of the membrane section. This centre can be easily determined by drawing lines 23 on the membrane section as shown in FIG. 2.

In a preferred format a second antibody technique is used. The antibody reacting specifically with the antigen being measured is the first antibody. Immobilization of this monoclonal anti-OA antibody directly on the membrane gave poor results.

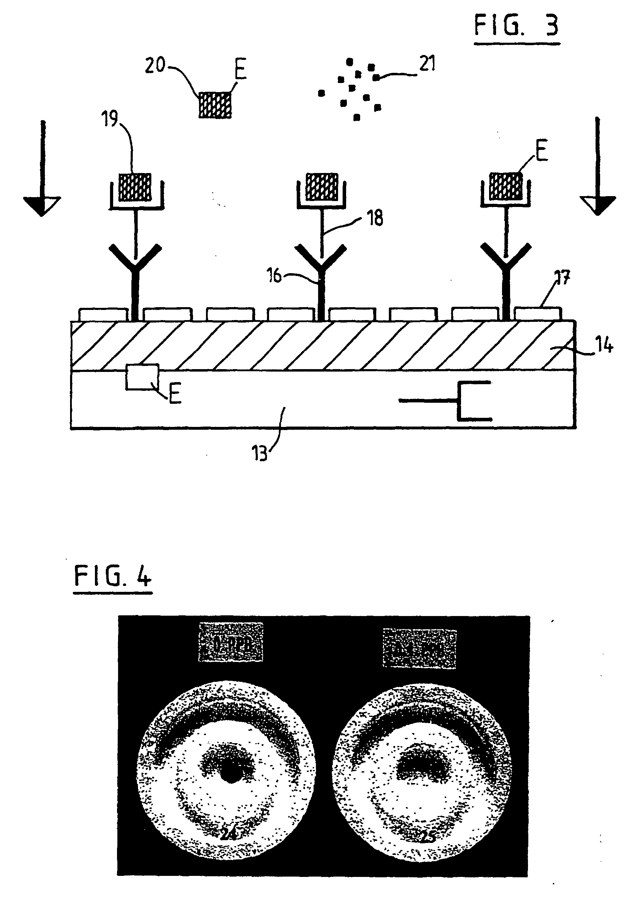

Concentrated solutions of anti-OA antibodies and OA-HRP had to be used to obtain some colour. Therefore a second antibody technique is used to overcome this problem. A second antibody is an antibody raised against the immunoglobulins of the animal species in which the first antibody was raised. The first anti-OA antibody is raised in mice, so the second antibody is an anti-mouse antibody and more specifically rabbit anti-mouse immunoglobulins (from Dako, Glostrup, Denmark; No. Z259; protein concentration 3.2 mg/ml; reacting with all mouse IgG subclasses, mouse IgA, and mouse IgM). In the second antibody technique undiluted rabbit anti-mouse immunoglobulins 16 (FIG. 3 [The arrows indicate the direction of reagent flow]) (3 µl) are spotted on the preferred membrane 14. The immunoreactants can be much more diluted (anti-OA antibody 1:50 and OA-HRP 1:50 instead of anti-OA antibody 1:1 and OA-HRP 1: 25). This can be explained as follows : for coating of an Immunodyne ABC membrane, a protein concentration of 1 to 10 mg/ml and a volume of between 1 and 3 µl are needed. The protein content of the original anti-OA immunoglobulin solution is 2.5 mg/ml. Thus the anti-OA would have to be used undiluted, which would be too expensive and which would result in a low sensitivity of the assay. Decreasing the anti-OA immunoglobulin and the OA-HRP concentration increases the assay sensitivity. Furthermore, it has been proved that antibodies immobilized by an antiglobulin retain full activity.

After coating the membrane with the rabbit anti-mouse immunoglobulins 16, the membranes are dried at 37°C for 30 minutes. Then, the remaining covalent sites of the entire membrane section are blocked. In this way, the undesired nonspecific binding is suppressed. Blocking is done by immobilizing on the membrane a protein which does not immunologically bind with any other component or reagent of the assay. Such protein, however, may be immunologically reactive towards components of another assay. Casein 17 is recommended by Pall BioSupport as blocking agent for the Immunodyne ABC membrane. Using casein 17 results in minimal background colour. So, the membranes are immersed in PBS (pH 7.4) containing 2% (wt/vol) casein sodium salt (Sigma Chemical Co.) for 30 minutes.

After being dried at 37 °C for 30 minutes and at room temperature for 30 minutes, the anti-mouse-coated membranes are stored in plastic vacuum-sealed bags at room temperature in the dark.

Assay procedure. To perform the test, the plastic bottom member 11 is filled with 1.5 g 100% cotton wool 13. The membrane 14 which is coated with the anti-mouse immunoglobulins 16 as previously described, is placed on the absorbent material 13.

The top member 10 is fitted on the bottom member 11 which is filled with the absorbent member and the membrane. The membrane 14 is placed on the absorbent pad in such a way that the centre 15 of the membrane where the antibody spot is located, is overlied by the aperture 12 of the top member 10. In this way assay reagents are directed through the aperture 12 to the antibody spot. The diameter of the aperture 12 (± 1 cm) is bigger than the diameter of the antibody spot (± 4 mm).

This antibody spot is not visible after coating and drying but becomes apparent after colour reaction.

When the test device is ready, assay reagents are successively dropped on the membrane 14 by using a micropipette.

First, 300 μl wash solution (PBS/0.05% Tween 20) is dropped onto the membrane 14. The wash solution is used as a prewetting agent to obtain an even flow of reagents through the membrane. It takes ±1 minute and 40 sec for the wash solution to flow through the membrane into and by the absorbent material. When some fluid remains on the edges of the aperture 12, it must be sucked up with a pipette and dropped again on the membrane 14.

The next step is adding 100 μl anti-OA immunoglobulin solution 18 (diluted 1:50 in PBS/0.05% Tween 20/0.1% BSA [assay buffer]). This first antibody will bind to the immobilized anti-mouse antibody 16 during passage through the membrane. When all the liquid is absorbed by the absorbent pad, the next reagent can be added: 300 μl wash solution to flush the unbound anti-OA immunoglobulin from the membrane 14 and into the absorbent material 13.

When the membrane appears dry, 600 μl OA standard solution (in 27% methanol/PBS/0.05%Tween 20) or sample extract solution is dropped onto the membrane. When OA antigens 19 are present in the sample or standard solution, they will be bound by the anti-OA immunoglobulins 18.

After passage of this solution through the membrane, 100 μl OA-HRP 20 (diluted 1:50 in PBS/0.05% Tween 20/0.1% BSA) is added. This will bind to anti-OA immunoglobulins 18 that have not yet bound antigens 19. As a consequence, when the antigen concentration in the sample or standard solution increases, there will be less OA-HRP bound. When no antigens are present, the amount of OA-HRP bound will be maximal. The next step is adding 600 μl wash solution to wash unbound enzyme conjugate into the absorbent layer 13.

Incubation times are extremely short. The immunochemical reaction between the components in the test fluids and the substances immobilized on the membrane takes place during the period of time in which the fluid is in contact with the reactive sites on the membrane i.e. during passage through the membrane.

Finally, 50 μl of substrate-chromogen solution 21 ($H_2O_2$-3,3',5,5'-tetramethylbenzidine [TMB]) is dropped onto the membrane. The immunochemically bound HRP 20 will convert the substrate to a coloured product.

After 1 minute the dot colour intensity is evaluated.

The whole assay procedure is performed at room temperature. It takes less than 15 minutes to perform the flow-through assay.

This immunoassay is a sequential competitive enzyme immunoassay. The method is based on the competition of the OA-HRP with the OA present in the test sample or standard solution for the anti-OA antibody on the membrane. Sequential means that the antigen is added first and the enzyme conjugate later. It is also possible to make use of a competitive enzyme immunoassay where antigen and enzyme conjugate are added together. As a result antigen and enzyme-labelled antigen compete simultaneously for the antibody on the solid phase. The decrease in colour intensity when antigens are present is more pronounced in the sequential method. A short second incubation period in the sequential method prevents the system approaching equilibrium.

A chromogenic substrate that makes low concentrations of peroxidase visible is needed. The commonly used soluble TMB substrate is preferred because it gives a high colour intensity with low concentrations of immunoreactants. TMB has no mutagenic or carcinogenic properties. The colourless TMB becomes blue when oxidized. The substrate-chromogen solution is prepared as described by Dürsch and Meyer (1992. Food Agric. Immunol. 4:211-218) with little modification. This solution is made prior to the assay by mixing equal volumes of solutions A and B.

Solution A (pH 5.0) contains 1.00 g of urea hydrogen peroxide (Sigma Chemical Co.; containing approximately 35% $H_2O_2$) per liter, 18.00 g of $Na_2HPO_4.2H_2O$ per liter, 10.30 g of citric acid.$H_2O$ per liter, and 0.05% Proclin 300 (Supelco, Inc., Bellefonte, PA, USA) as preservative. Solution B (pH 2.4) contains 500 mg of TMB (Sigma Chemical Co.), 40 ml of dimethyl sulfoxide, 960 ml water of HPLC grade, 10.30 g of citric acid.$H_2O$, and 0.05 % Proclin 300. Solution A and B are stored in the dark. Proclin 300 is also added as preservative to all the buffers used in the assay because Proclin 300 has no effect on the HRP enzyme activity and it is suitable for use with antibody-based diagnostic assays.

In the assay the anti-OA antibody and the OA-HRP are both diluted 1:50. Determination of the optimal antibody and conjugate dilution is the first step in developing an enzyme immunoassay. The purpose of this flow-through assay is to allow visual evaluation. It is more difficult to see differences between two dark colours than between two pale colours or between no colour and a pale colour. The colour intensities of the spots must be high enough to be seen and low enough to enable observation of differences in colour intensity. When the working dilutions are high, the assay is more sensitive. However, when the working dilutions are too high the colour will be too pale. The optimal dilutions for the immunoreagents were determined by trial and error. A dilution of 1:50 for the anti-OA immunoglobulin and 1:50 for the OA-HRP results in an intense blue dot when no antigens are present in the test sample. With these dilutions, it is also possible to make a visual distinction between very small changes in colour intensity.

The colour intensity can be visually evaluated i.e. with the naked eye. This is done by comparing the dot colour intensity of the test membrane with that of the negative control. A negative control sample contains no antigens. The negative control test is simultaneously performed with the assays for the OA determination. The procedure for the negative control is the same as mentioned before but instead of 600 µl test sample, 600 µl of 27% methanol/PBS/0.05% Tween 20 is dropped on the membrane.

It results in the most intense blue colour because of the inverse relationship between toxin concentration and colour development.

When a blue coloured dot, even less intensive than the colour of the negative control, appears on the test membrane, the sample is considered to be negative for OA. If no colour appears on the test membrane, it indicates that the test sample contains sufficient antigen to saturate the antibody and the sample is considered to be positive for OA. If no colour appears on the negative control membrane, the test must be considered as invalid.

Since the colour is not stable for long, the reading must be done immediately.

Visual evaluation of colour in terms of colour/no colour or more intensive/less intensive colour is easy, but expressing a colour accurately and describing that colour to another person is very difficult for the human eye. A variety of conditions affects how a colour looks: light-source, observer, size, background and direction. With a colorimeter, it is possible to quantify colour and express it numerically, according to international standards. Colorimeters have sensitivities corresponding to those of the human eye, but because they always take measurements using the same light-source and illumination method, the measurement conditions will always be identical, regardless of whether it is day or night, indoors or outdoors. A preferred colorimeter is the Chroma Meter CR-321 (Minolta). Details about this colorimeter are described by Minolta Co., Ltd. (1994. Precise color communication.). The principle on which the colorimetric evaluation is based, is called the tristimulus method. It measures light in a way similar to how the human eye perceives light and thus it is not a spectrophotometric method. The L*a*b* colour space is used for the measurements of the dots on the membranes. It is one of the uniform colour spaces defined by an international organization concerned with light and colour, the Commission Internationale de l'Eclairage (CIE), in 1976. A colour is expressed by 3 values : L* indicates lightness, a* and b* are the chromaticity coordinates. These a* and b* indicate colour directions : +a* is the red direction, -a* is the green one, +b* is the yellow and -b* is the blue direction. For the flow-through test, the difference between the colour of a white membrane (as a reference) and the dot colour intensity on the test membrane is measured. In the L*a*b* colour space this colour difference is expressed as a single numerical value, $\Delta E^*_{ab}$.

A test kit for the detection of OA is provided which comprises the aforementioned test devices consisting of plastic top 10 and bottom member 11, absorbent material 13, membrane 14 coated with anti-mouse immunoglobulins 16, wash solution, lyophilized anti-OA immunoglobulins 18, assay buffer, negative control solution, lyophilized OA-HRP 20 and substrate-chromogen solutions A and B 21. All materials and reagents are supplied in quantities sufficient for performing a predefined number of assays.

EXAMPLE 1

A flow-through enzyme immunoassay is performed for the detection of OA in buffer solutions. Therefore, a stock solution of OA (1 mg/ml) is prepared in methanol and stored at -20°C. The OA is purchased from Sigma Chemical Co. Methanol is HPLC grade. A working stock solution of OA (100 µg/ml) is prepared in 10% methanol/PBS and stored at ≤ 5°C. Working standards (0.2 ng/ml, 0.3 ng/ml, 0.4 ng/ml and 0.6 ng/ml) are prepared on the day of assay in 27% methanol/PBS/0.05% Tween 20. All OA solutions are kept in the dark. The glassware and OA waste are decontaminated by keeping them 24 hours in sodium hypochlorite solution (household bleach).

Five assays are performed simultaneously to create a dose-response curve for OA. Five test devices comprising top member 10 and bottom member 11, absorbent member 13 and membrane 14 coated with anti-mouse immunoglobulins 16 are used.

The assembling of the test device has been previously described.

The same assay procedure as described above, is used. To perform 5 assays simultaneously, the following must be done : the correct volume of an assay reagent is dropped on the membrane of the first test device, followed by dropping the same volume of the same immunoreagent on the membrane of the second, third, fourth and fifth test device. When the membrane of the first test device appears dry, the next reagent can be added. This reagent must be dropped on the second membrane when this appears dry too. The same is done with the third, fourth and fifth test device.

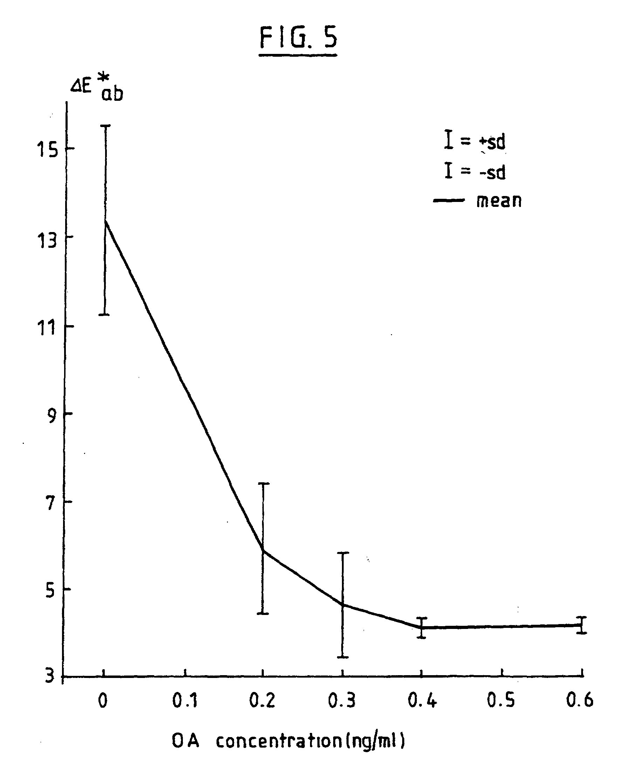

Standard solutions are added by dropping 600 µl of an OA solution of 0 ng/ml, 0.2 ng/ml, 0.3 ng/ml, 0.4 ng/ml, and 0.6 ng/ml on the first, second, third, fourth, and fifth test device, respectively. After colour reaction the colour on the 0 ng/ml test membrane will be most intensive. There is an inverse relationship between toxin concentration and colour development. The smallest toxin concentration that results in a complete suppression of the colour is considered the visual detection limit. For this flow-through enzyme immunoassay the visual detection limit is 0.4 ppb (0.4 ng of OA per ml of buffer solution). FIG. 4 shows the negative control (0 ppb) resulting in an intense colour 24 and the visual detection limit (0.4 ppb) resulting in no colour development 25.

By using the Minolta Chroma Meter CR-321, a dose-response curve for OA could be established (FIG. 5). This was obtained by plotting the OA concentration against the difference ($\Delta E^*_{ab}$) between the colour of a white membrane (as a reference) and the dot colour intensity of the test membrane. The experiment was repeated 7 times on different days with membranes coated on different days. Means and standard deviations were calculated and are shown in FIG. 5. Coefficients of variation (CVs) are between 4 and 26%. This can be attributed to variations in the coating and incubation steps. FIG. 5 shows that $\Delta E^*_{ab}$ never reaches zero. This is due to background colour development caused by light, air and time. However, this background colour development is minimal, and the difference between dot and background colours remains very large. FIG. 5 shows an important decrease in dot colour intensity between 0 and 0.4 ppb. From 0.4 ppb there is a plateau with only very little background colour.

EXAMPLE 2

The flow-through enzyme immunoassay can be used for the detection of OA in wheat.

The wheat sample is milled in a coffee grinder. A 5g portion is mixed with 15 ml of 80% methanol-water for 1 hour. Methanol and water are HPLC grade. The suspension is filtered through Whatman no. 4 paper. Before the assay, the filtrate is diluted 3 times with PBS/0.05% Tween 20. This dilution is filtered through a 0.45 µm filter (Chromafil disposable filter, Macherey-Nagel, Düren, Germany). This filtrate (600 µl) is used in the assay.

This extraction and clean-up procedure is just an example. Other procedures can be used as well. This extraction and sample pretreatment are very simple. Methanol in combination with water is often used for extraction of OA from cereals. In this assay wheat is extracted with 80% methanol-water. Because it is not possible to use such a high methanol content in an enzyme immunoassay, the filtrate is diluted 3 times.

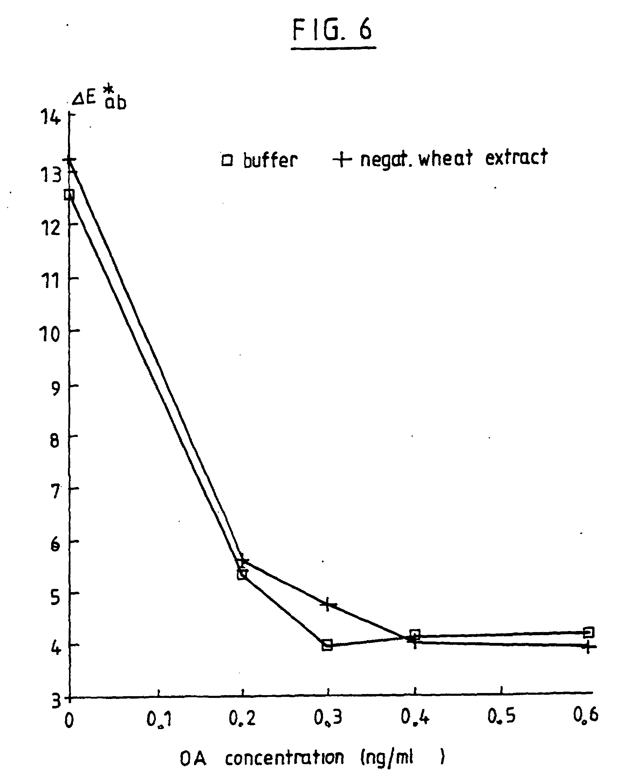

This results in a methanol content of 27% which does not affect the dot colour development. When diluting the extract, precipitates are formed. These are removed by filtering this turbid dilution through a 0.45 µm filter. As a result the liquid flows easily through the membrane and the membrane remains white. There is no matrix interference. This is shown in FIG. 6. The x axis indicates the OA concentration, and the y axis indicates the difference ($\Delta E^*_{ab}$) between the colour of a white membrane (as a reference) and the dot colour intensity on the test membrane. OA standards were prepared in buffer solution (27% methanol/PBS/0.05% Tween 20) and in OA-free wheat extract. The assay was repeated 3 times on different days. Each point represents the mean of 3 replicates. CVs are between 2 and 20%. The negative wheat sample was extracted on the day of assay and the OA standard solutions were freshly prepared every day. The standards diluted in buffer solution and in the negative wheat extract produce identical curves which superimpose. This means that the matrix components-do not interfere with the immunological reactions.

Using this extraction procedure spiked wheat samples are tested. Spiked samples are prepared one day prior to extraction. Therefore the appropriate volume of an OA-methanol solution (1.25 ng/µl: concentration determined by UV spectrometry) is added to 5g of ground wheat. After 1 day the methanol is evaporated. The spiked samples are shaken before extraction.

Due to the proposal of the European Commission (maximum permitted level of 4 µg/kg for OA in cereals) it must be possible to detect the presence of 4 µg OA/kg wheat. With the aforementioned extraction method and flow-through assay procedure a wheat sample spiked with 4 µg/kg results in a complete colour suppression. As a result, when there is no dot colour development, the sample is considered to be positive for OA. This result must be confirmed by methods other than immunoassays. When looking at the literature this is the first field test for OA that reaches this sensitivity.

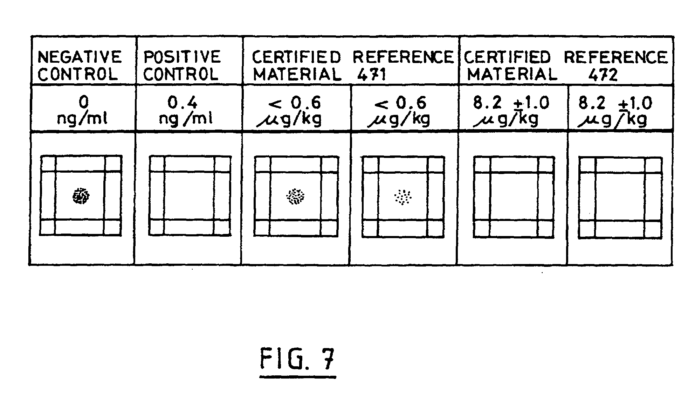

To check the developed flow-through assay, certified reference materials (European Commission, Joint Research Centre, Institute for Reference Materials and Measurements [IRMM], Geel, Belgium) have been used : wheat reference material RM 471 (certified OA mass fraction < 0.6 µg/kg) and RM 472 (certified OA mass fraction = 8.2±1.0 µg/kg). FIG. 7 shows the results. CRM 471 always (n=6) resulted in a colour intensity similar to that of the negative control. A positive control (0.4 ng/ml) was done as well. CRM 472 always (n=6) resulted in no dot colour development and a $\Delta E^*_{ab}$ value similar to that of the positive control.

TABLE 1.

| Some important naturally occurring mycotoxins, the toxin producing moulds, the major toxic effects in humans and animals and their typical natural substrates. | | | |
|---|---|---|---|
| **MYCOTOXINS** | **MAJOR PRODUCING FUNGI** | **MAJOR TOXIC EFFECTS** | **TARGET COMMODITY** |
| AFLATOXINS | Aspergillus flavus A. parasiticus | hepatotoxic, carcinogenic, mutagenic, immunosuppressive, teratogenic | nuts, cereals, figs, milk (products), cottonseed |

EP 0 893 690 B1

TABLE 1.   (continued)

| Some important naturally occurring mycotoxins, the toxin producing moulds, the major toxic effects in humans and animals and their typical natural substrates. | | | |
|---|---|---|---|
| **MYCOTOXINS** | **MAJOR PRODUCING FUNGI** | **MAJOR TOXIC EFFECTS** | **TARGET COMMODITY** |
| CITRININ | Penicillium citrinum | nephrotoxic | cereals, rice |
| FUMONISINS | Fusarium moniliforme | neurotoxic, hepatotoxic, carcinogenic | cereals (maize) |
| OCHRATOXINS | A. ochraceus P. verrucosum | carcinogenic, genotoxic, immunosuppressive, nephrotoxic, teratogenic | cereals, coffee, beer, animal feed, pig kidney |
| PATULIN | P. expansum | mutagenic, neurotoxic | apple(juice) |
| TRICHOTHECENES (e. g. T-2 toxin. deoxynivalenol) | various species of Fusarium | immunosuppressive, alimentary toxic aleukia (ATA), neurotoxic, dermatotoxic | cereals, rice |
| ZEARALENONE | F. graminearum | estrogenic, anabolic | cereals |

## Claims

1.  Ftow-through immunoassay for detecting a mycotoxin (19) chosen from the group of mycotoxins comprising aflatoxins, citrinin, fumonisins, ochratoxins, patulin, trichothecenes and zearalenones, using a container containing an absorbent material and having an opening closed by a microporous surface-modified hydrophilic membrane contacting the absorbent material and having pores with a size lower than 1 $\mu$m and which has a first part that is coated with a first antibody (16) which is immunologically reactive with an antibody (18) against the mycotoxin (19) to be detected and a second part on which proteins which are neither immunologically reactive with said first antibody nor with the mycotoxin to be detected are immobilized,

    and whereby the assay comprises the following steps in order of execution, whereby a subsequent step is only executed after the solution used in the previous step is adsorbed by the absorbent material (13):

    -   flowing through the membrane (14) of a wash solution,
    -   flowing through the membrane of a solution of an antibody (18) against the mycotoxin to be detected,
    -   flowing through the membrane (14) of a wash solution so as to flush from the membrane antibody (18) which is not bound to the first antibody (16) on the membrane,
    -   flowing through the membrane (14) of the sample extract solution,
    -   flowing through the membrane of a solution of an enzyme-labeled antigen (20) which is a conjugate of the mycotoxin to be detected with an enzyme,
    -   flowing through the membrane (14) of a wash solution,
    -   flowing through the membrane (14) of a solution (21) of a substrate-chromogen causing a color development upon reaction with the enzyme.

2.  Flow-through immunoassay according to claim 1, whereby the enzyme is horseradish peroxidase (HRP).

3.  Flow-through immunoassay according to claim 1 or 2, whereby the substrate-chromogen is $H_2O_2$-3,3',5,5'-tetramethylbenzidine (TMB).

4.  Flow-through immunoassay according to any of claims 1 to 3, whereby the color development is evaluated visually or measured by means of a colorimeter.

5.  Test kit for performing a flow-through immunoassay for detecting a mycotoxin (19) chosen from the group of mycotoxins comprising aflatoxins, citrinin, fumonisins, ochratoxins, patulin, trichothecenes and zearalenones, in a sample extract solution, said test kit comprising at least:

- a device comprising a bottom member (11) forming a reservoir comprising absorbent material (13) and a top member (10) which fits the bottom member (11) and which presents a single opening (12) through which reagents can be dropped,
- a microporous surface-modified hydrophilic membrane (14) placed on the absorbent material, said membrane having pores with a size lower than 1 μm and which is coated with a first antibody (16) which is immunologically reactive with an antibody (18) which has been raised against the mycotoxin to be detected,
- a wash solution,
- an assay buffer,
- a solution of the antibody (18) against the mycotoxin to be detected,
- a solution of enzyme-labeled antigen (20), which is a conjugate of the mycotoxin to be detected with an enzyme,
- a solution of substrate-chromogen which causes a color development upon reaction with the enzyme, and
- a negative control.

**6.** A test kit according to claim 5, further **characterized in that** the membrane (14) contains lines (23) for determining the center of said membrane.

**7.** A test kit according to claim 5 or 6, further **characterized in that** said absorbent material (13) in the device is cotton wool.

**8.** Use of a test kit according to any of claims 5 to 7 for detecting Ochratoxin A in a sample extract wherein in said test kit, the solution (18) is a solution of the antibody against Ochratoxin A and the solution (20) is a solution of enzyme-labeled Ochratoxin A (20), which is a conjugate of Ochratoxin A with an enzyme.

**Patentansprüche**

**1.** Durchfluss-Immunoassay für das Erfassen eines Mykotoxins (19), das aus der Gruppe von Mykotoxinen ausgewählt ist, die Aflatoxine, Citrinin, Fumonisine, Ochratoxine, Palutin, Trichothecene und Zearalenone umfassen, unter Zuhilfenahme eines Behälters, der ein absorptionsfähiges Material enthält und eine Öffnung aufweist, die durch eine mikroporenhaltige, oberflächenmodifizierte hydrophile Membran geschlossen ist, die das absorptionsfähige Material kontaktiert und Poren einer Größe von weniger als 1 μm aufweist, und die einen ersten Teil aufweist, der mit einem ersten Antikörper (16) beschichtet ist, der immunologisch reaktiv ist mit einem Antikörper (18) gegen das zu erfassende Mykotoxin (19) sowie einen zweiten Teil, auf dem Proteine immobilisiert sind, die weder mit dem ersten Antikörper noch mit dem zu erfassenden Mykotoxin immunologisch reaktiv sind, und wobei der Assay folgende Schritte in der Reihenfolge der Ausführung umfasst, wobei ein darauffolgender Schritt nur ausgeführt wird, nachdem die im vorhergehenden Schritt verwendete Lösung durch das absorptionsfähige Material (13) adsorbiert ist:

- Hindurchfließen einer Waschlösung durch die Membran (14),
- Hindurchfließen einer Lösung eines Antikörpers (18) gegen das zu erfassende Mykotoxin durch die Membran,
- Hindurchfließen einer Waschlösung durch die Membran (14) derart, dass Antikörper (18), der nicht an den ersten Antikörper (16) auf der Membran gebunden ist, aus der Membran hinausgespült wird,
- Hindurchfließen der Probenextraktlösung durch die Membran (14)
- Hindurchfließen, durch die Membran, einer Lösung eines enzymmarkierten Antigens (20), bei dem es sich um ein Konjugat des zu erfassenden Mykotoxins mit einem Enzym handelt,
- Hindurchfließen einer Waschlösung durch die Membran (14)
- Hindurchfließen, durch die Membran (14), einer Lösung (21) eines Substratchromogens, das auf die Reaktion mit dem Enzym hin eine Farbentwicklung hervorruft.

**2.** Durchfluss-Immunoassay nach Anspruch 1, wobei es sich bei dem Enzym um Meerrettichperoxidase (MRP) handelt.

**3.** Durchfluss-Immunoassay nach Anspruch 1 oder 2, wobei es sich bei dem Substratchromogen um H2O2-3,3',5,5'-tetramethylbenzidin (TMB) handelt.

4. Durchfluss-Immunoassay nach einem der Ansprüche 1 bis 3, wobei die Farbentwicklung visuell beurteilt oder durch ein Kolorimeter gemessen wird.

5. Testkit für das Durchführen eines Durchfluss-Immunoassays für das Erfassen eines Mykotoxins (19), das aus der Gruppe von Mykotoxinen ausgewählt ist, die Aflatoxine, Citrinin, Fumonisine, Ochratoxine, Palutin, Trichothecene und Zearalenone umfassen, in einer Probenextraktlösung, wobei der Testkit mindestens Folgendes umfasst:

   - ein Gerät umfassend ein unteres Glied (11), das ein absorptionsfähiges Material (13) umfassendes Reservoir bildet, und ein oberes Glied (10), das auf das untere Glied (11) passt und das eine einzige Öffnung (12) bietet, durch die Reagentien hindurchgetropft werden können,
   - eine mikroporenhaltige oberflächenmodifizierte hydrophile Membran (14), die auf das absorptionsfähige Material aufgelegt ist, wobei die Membran Poren einer Größe von weniger als 1 μm aufweist und mit einem ersten Antikörper (16) beschichtet ist, der mit einem Antikörper (18) immunologisch reaktiv ist, der gegen das zu erfassende Mykotoxin gezüchtet worden ist,
   - eine Waschlösung,
   - einen Assaypuffer,
   - eine Lösung des Antikörpers (18) gegen das zu erfassende Mykotoxin,
   - eine Lösung von enzymmarkiertem Antigen (20), bei dem es sich um ein Konjugat des zu erfassenden Mykotoxins mit einem Enzym handelt,
   - eine Lösung von Substratchromogen, das auf die Reaktion mit dem Enzym hin eine Farbentwicklung hervorruft und
   - eine negative Kontrolle.

6. Testkit nach Anspruch 5, des Weiteren **dadurch gekennzeichnet, dass** die Membran (14) Linien (23) für das Bestimmen der Mitte der genannten Membran enthält.

7. Testkit nach Anspruch 5 oder 6, des Weiteren **dadurch gekennzeichnet, dass** es sich bei dem absorptionsfähigen Material (13) in dem Gerät um Baumwolle handelt.

8. Verwendung eines Testkits nach einem der Ansprüche 5 bis 7 für das Erfassen von Ochratoxin A in einem Probenextrakt, wobei in dem Testkit die Lösung (18) eine Lösung des Antikörpers gegen Ochratoxin A ist und die Lösung (20) eine Lösung des enzymmarkierten Ochratoxins A (20) ist, bei dem es sich um ein Konjugat von Ochratoxin A mit einem Enzym handelt.

## Revendications

1. Dosage immunologique en flux continu permettant de détecter une mycotoxine (19) choisie parmi le groupe de mycotoxines comprenant les aflatoxines, la citrinine, les fumonisines, les ochratoxines, la patuline, les trichothécènes et les zéaralénones, en utilisant un récipient contenant un matériau absorbant et ayant une ouverture fermée par une membrane hydrophile microporeuse à surface modifiée en contact avec le matériau absorbant et ayant des orifices présentant une taille inférieure à 1 μm et qui a une première partie recouverte avec un premier anticorps (16) qui est immunologiquement réactif avec un anticorps (18) contre la mycotoxine (19) devant être détectée et une deuxième partie sur laquelle des protéines qui ne sont immunologiquement réactives ni avec ledit premier anticorps ni avec la mycotoxine devant être détectée, sont immobilisées, et dans lequel le dosage comprend les étapes suivantes par ordre d'exécution, dans lequel une étape subséquente est uniquement réalisée une fois la solution utilisée dans l'étape précédente adsorbée par le matériau absorbant (13) :

   - circulation à travers la membrane (14) d'une solution de lavage,
   - circulation à travers la membrane d'une solution d'un anticorps (18) contre la mycotoxine devant être détectée,
   - circulation à travers la membrane (14) d'une solution de lavage de façon à rincer de la membrane l'anticorps (18) qui n'est pas lié au premier anticorps (16) sur la membrane,
   - circulation à travers la membrane (14) de la solution d'extrait d'échantillon,
   - circulation à travers la membrane d'une solution d'un antigène marqué par une enzyme (20) qui est un conjugué de la mycotoxine devant être détectée et d'une enzyme,
   - circulation à travers la membrane (14) d'une solution de lavage,
   - circulation à travers la membrane (14) d'une solution (21) d'un substrat chromogène provoquant un développement de couleur lors de la réaction avec l'enzyme.

**2.** Dosage immunologique en flux continu selon la revendication 1, dans lequel l'enzyme est la peroxydase de raifort (HRP).

**3.** Dosage immunologique en flux continu selon la revendication 1 ou 2, dans lequel le substrat chromogène est $H_2O_2$-3,3',5,5'-tétraméthylbenzidine (TMB).

**4.** Dosage immunologique en flux continu selon l'une quelconque des revendications 1 à 3, dans lequel le développement de couleur est évalué de façon visuelle ou est mesuré à l'aide d'un colorimètre.

**5.** Trousse d'essai permettant de réaliser un dosage immunologique en flux continu afin de détecter une mycotoxine (19) choisie parmi le groupe des mycotoxines comprenant les aflatoxines, la citrinine, les fumonisines, les ochratoxines, la patuline, les trichothécènes et les zéaralénones, dans une solution d'extrait d'échantillon, ladite trousse d'essai comprenant au moins :

- un dispositif comprenant une pièce inférieure (11) formant un réservoir comprenant le matériau absorbant (13) et une pièce supérieure (10) s'ajustant à la pièce inférieure (11) et présentant une seule ouverture (12) à travers laquelle les réactifs peuvent être versés,
- une membrane hydrophile microporeuse à surface modifiée (14) placée sur le matériau absorbant, ladite membrane ayant des orifices présentant une taille inférieure à 1 μm et ayant une première partie recouverte avec un premier anticorps (16) qui est immunologiquement réactif avec un anticorps (18) contre la mycotoxine (19) devant être détectée,
- une solution de lavage,
- un tampon d'analyse,
- une solution d'anticorps (18) contre la mycotoxine devant être détectée,
- une solution d'antigène marqué par une enzyme (20), qui est un conjugué de la mycotoxine devant être détectée et d'une enzyme,
- une solution de substrat chromogène provoquant un développement de couleur lors de la réaction avec l'enzyme, et
- un témoin négatif.

**6.** Trousse d'essai selon la revendication 5, **caractérisée de plus en ce que** la membrane (14) comprend des lignes (23) permettant de déterminer le centre de ladite membrane.

**7.** Trousse d'essai selon la revendication 5 ou 6, **caractérisée de plus en ce que** ledit matériau absorbant (13) situé dans le dispositif est de l'ouate.

**8.** Utilisation d'une trousse d'essai selon l'une quelconque des revendications 5 à 7, permettant de détecter l'Ochratoxine A dans un extrait d'échantillon dans laquelle dans ladite trousse d'essai, la solution (18) est une solution d'anticorps contre l'Ochratoxine A et la solution (20) est une solution d'Ochratoxine A marquée par une enzyme (20), qui est un conjugué d'Ochratoxine A et d'une enzyme.

FIG. 1

FIG. 2

FIG. 8

FIG. 3

FIG. 4

# FIG. 5

# FIG. 6

| NEGATIVE CONTROL | POSITIVE CONTROL | CERTIFIED REFERENCE MATERIAL 471 | | CERTIFIED REFERENCE MATERIAL 472 | |
|---|---|---|---|---|---|
| 0 ng/ml | 0.4 ng/ml | < 0.6 $\mu$g/kg | < 0.6 $\mu$g/kg | 8.2 ±1.0 $\mu$g/kg | 8.2 ±1.0 $\mu$g/kg |

FIG. 7